# EUROPEAN PATENT APPLICATION

(11) **EP 2 301 454 A2**
(43) Date of publication of application: **30.03.2011**
(21) Application number: 10009659.3
(22) Date of filing: 16.09.2010
(51) Int. Cl.: A61B 17/34

(54) **Organ fixture and gastric fistula catheter set**

(30) Priority: 24.09.2009 JP 2009218774
(71) Applicant: Tyco Healthcare Group LP, Mansfield, MA 02048 (US)
(72) Inventor: Nagata, Katsuki, Fukuroi Shizuoka 437-0004 (JP)
(74) Representative: Olsson, Carl H.S.

(57) **Abstract**

An organ-fixing instrument and a gastric fistula catheter able to accomplish proper suturing reliably and through a simple procedure.

An organ-fixing instrument 10 is structured by a transversely elastic, cylindrical sheath 12, and a body surface installation part 11 attached to the base part of the sheath 12. In the body surface installation part 11 there are provided a through-hole 13 communicating with the sheath 12 and a pair of needle through-holes 14, 15. The sheath 12 is structured by a web-shaped covering part 16 made from a resin, a plurality of reinforcing parts 17 provided at the base end portion of the covering part 16, and skeletal parts 18a, 18b provided at the tip portion of the covering part 16. A suturing thread catch 18 projected in an axial direction by compression in the axial direction is structured by the tip side portion of the covering part 16 and the skeletal parts 18a, 18b.

## Description

### FIELD OF THE DISCLOSURE

The present disclosure concerns an organ-fixing instrument and gastric fistula catheter set used when fixing a predetermined organ to a skin wall region with suturing thread in the body of the patient.

### BACKGROUND OF THE DISCLOSURE

Conventionally, an organ-fixing instrument has been used to fix a predetermined organ to a skin wall region with suturing thread in the body of a patient. For example, a gastric fistula catheter has been used to supply fluid food, nutrient agents, or other such fluid food and drink to individuals with diminished ability to consume food through the mouth on their own due to old age or illness; however, such a gastric fistula catheter is installed by forming a hole in the abdomen of the patient. In such an instance, an organ-fixing instrument is used to fix the abdominal wall and gastric wall together in advance in order to install a gastric fistula catheter properly.

This organ-fixing instrument (medical instrument) is equipped with two puncture needles disposed parallel to each other to maintain spacing, and when used to fix the stomach, first, the two puncture needles are used to pierce the interior of the stomach simultaneously from the skin surface of the body of the patient. Next, a suturing thread is passed through one puncture needle; a stylet equipped with a rod-shaped member and an annular member secured to the tip of the rod-shaped member is passed through the other puncture needle; and the stylet is withdrawn from the puncture needle in a state wherein the suturing thread is grasped by the annular member inside the stomach. The two puncture needles are then withdrawn from the patient, and fixation of the stomach to the skin wall region is completed by tying both end-portions of the suturing thread protruding from the body of the patient.

With a conventional organ-fixing instrument, a suturing thread must be grasped by an annular member within the stomach, and for this purpose, a stylet must be inserted into a puncture needle skillfully, and the protruding direction and shape of the annular member must be correct. But a problem exists in that this procedure is difficult, it is consequently difficult to make the annular member grasp the suturing thread, and reliability is low.

The present invention was created in order to address such problems and has an object of offering an organ-fixing instrument and gastric fistula catheter set able to accomplish proper suturing reliably and through a simple procedure.

### SUMMARY OF THE DISCLOSURE

To achieve the aforementioned object, the structural characteristics of the organ-fixing instrument pertaining to the present invention are an organ-fixing instrument equipped with a transversely elastic cylindrical sheath and a body surface installation part attached to the base end part of the sheath, used when fixing a predetermined organ to the skin-side wall area with suturing thread in the body of a patient; wherein the body surface insulation part is equipped with a through-hole communicating with the sheath, and with a pair of needle through-holes enclosing the through-hole and formed at both sides of the through-hole and allowing passage of a puncture needle approximately parallel to the sheath; and wherein the sheath is equipped with a suturing thread catch which is provided at the tip side of the sheath and projected in a transverse direction by application of a force which tends to bring both ends closer to each other along an axial direction and which is deformed when the puncture needles pass into the pair of needle through-holes until said puncture needles reach a puncturable location.

### BRIEF DESCRIPTION OF THE DRAWINGS

(FIGURE 1) Figure 1 illustrates the organ-fixing instrument pertaining to one preferred embodiment of the present invention; in the figure, (a) is a planar view, and (b) is a frontal view.
(FIGURE 2) Figure 2 is a cross-sectional view illustrating a state in which the suturing thread catch of a sheath projects in a transverse direction.
(FIGURE 3) Figure 3 is a frontal view illustrating a state in which an organ-fixing instrument is inserted into a hole part along a guide wire passed through the hole part.
(FIGURE 4) Figure 4 is a partial frontal view illustrating a cross-section of part of a state in which a dilator is inserted into an organ-fixing instrument, and the diameter of a sheath and hole part are enlarged.
(FIGURE 5) Figure 5 is a frontal view illustrating a dilator.
(FIGURE 6) Figure 6 is a frontal view illustrating a gastric fistula catheter; in the figure, (a) is a planar view, and (b) is a frontal view.
(FIGURE 7) Figure 7 is a frontal view illustrating a state in which a gastric fistula catheter is extended by an extender.
(FIGURE 8) Figure 8 is a frontal view illustrating a cross-section of part of a state in which an extended gastric fistula catheter is inserted into an organ-fixing instrument.
(FIGURE 9) Figure 9 illustrates a state in which a suturing thread catch has been extended in a transverse direction by restoring a gastric fistula catheter to its original state; in the figure (a) is a frontal view illustrating a partial cross-section, and (b) is a bottom view.
(FIGURE 10) Figure 10 illustrates a state in which a pair of suturing needles into which a suturing thread has been passed are inserted from a body surface installation part and passed through a suturing thread catch; in the figure, (a) is a frontal view illustrating a partial cross-section, and (b) is a bottom view.
(FIGURE 11) Figure 11 is a frontal view illustrating a cross-section of a partial state in which a pair of puncture needles and an organ-fixing instrument have been withdrawn, and a suturing thread has been passed through the skin surface into an organ and made to project from the skin surface.
(FIGURE 12) Figure 12 is a frontal view illustrating a cross-section of a partial state in which implantation of a gastric fistula catheter and suturing of the stomach have been completed.

### DETAILED DESCRIPTION OF THE DISCLOSURE

In the organ-fixing instrument pertaining to the present invention and thus structured, at the body surface installation part, there are formed a through-hole communicating with the sheath, and a pair of needle through-holes allowing passage of a puncture needle, and at the tip side of the sheath, there is provided a suturing thread catch projected in a transverse direction by application of a force which tends to bring both ends closer to each other along an axial direction, and which is deformed until the puncture needles passing through the needle through-holes reach a puncturable location. For this purpose, holes passing form the skin surface side of the body of a patient into an organ are provided, and the suturing thread catch can be inserted from the skin surface side of the body of a patient into an organ and put into a state projecting in a transverse direction in the organ by passing the sheath of the organ-fixing instrument through these holes. In such an instance, the suturing thread catch is projected in a transverse direction by inserting a gastric fistula catheter or other such elastic item from the through-holes in the body surface installation part into the sheath and extending or contracting, etc. within the sheath.

When a pair of puncture needles in each of which a suturing thread has been inserted is then inserted from the pair of needle through-holes in the body surface installation part, and the tip thereof punctures the suturing thread catch and the suturing thread is thereby wrapped onto the suturing thread catch, the pair of suturing threads and the sheath assume a nearly U-shaped continuous form passing from the holes made by the puncture needles, into and through the organ, and through the holes where the sheath has been inserted. In this state, when the pair of puncture needles and organ-fixing instrument sheath are withdrawn from the body of the patient with the suturing threads remaining in the body of the patient, the tip portion of the pair of suturing threads projects from each hole made by the puncture needles, into and through the organ, from the holes where the sheath has been inserted, and to the skin surface side.

Thus, by tying both sides of each among the pair of suturing threads located on the skin side of the patient, the organ can be fixed to a skin layer of the patient with the pair of suturing threads. In this instance, the tip part of the suturing thread has been given a structure able to wrap onto the suturing thread catch. Thereby, simply by passing the pair of puncture needles, through each of which a suturing thread has been passed, through a pair of needle through-holes in the body surface installation part, the tip thereof punctures the suturing thread catch, and the suturing thread is wound onto the suturing thread catch by pulling the suturing thread in a state in which the puncture needles have been withdrawn from the suturing thread catch. The procedure to wrap the suturing thread onto the suturing thread catch is thereby facilitated, and the suturing thread is also wrapped onto the suturing thread catch reliably.

Additionally, because the sheath of the organ-fixing instrument has been formed with a transversely elastic, cylindrical shape, the sheath assumes a narrow rod-shape when inserted into the body of a patient, and once the sheath has been inserted into the body, the diameter of the sheath and the holes formed in the body of the patient can be enlarged by inserting a dilator into the sheath. Subsequent procedures are thereby facilitated; for example, installation of a gastric fistula catheter in the holes formed in the body. In this instance, installation of a gastric fistula catheter and fixation of the stomach can be performed simultaneously.

Another structural characteristic of the organ-fixing instrument pertaining to the present invention is that the body of the sheath is structured by a cover part comprising a tube made from a resin and equipped with a webbed locking part, and the suturing thread catch is structured by the provision at the tip of the covering part of a skeletal part with a hinged structure.

Because the body of the sheath is structured by a covering part comprising a tube made from a resin equipped with a webbed locking part, the suturing thread wraps easily onto the suturing thread catch. Examples which may be used as such a tube are a webbed tube comprising a web made from a resin, or a webbed tube incorporating a laminate resin and a web made from a resin. Additionally, skeletal parts may, for example, be structured by a pair of rod-shaped bodies or the like with a bend formed at the center in the lengthwise direction, and with the bend made to project toward the outside in each case when compression is applied in an axial direction. In this instance, the skeletal part is made to project not in the entire circumferential direction, but at a location puncturable by the pair of puncture needles, and the covering part is made to spread together with the skeletal part. This covering part may, for example, be given a structure made long and slender by pulling the base and tip parts in opposite directions, or a webbed, cylindrical body spread in a transverse direction by pulling in a circumferential direction. The skeletal part is also structured by an item which bends when a sheath endowed with plasticity is withdrawn to the outside of the body.

Yet another structural characteristic of the organ-fixing instrument pertaining to the present invention is that a reinforcing part is provided extending along an axial direction at the base region of the covering part. The strength of the base end portion of the sheath located at holes is thereby greatly increased when the sheath is inserted into holes formed in the body of a patient. Any occurrence of misalignment between the holes provided at the skin side of the body and the holes provided in the organ is thus prevented. The item used as a reinforcing part in this instance is a plurality of rod-shaped members disposed to maintain spacing in the circumferential direction of the sheath, or another such item not impeding extension and contraction of the sheath in a transverse direction. The reinforcing part may also comprise an item endowed with plasticity and able to bend when the sheath is withdrawn from the body.

Yet another structural characteristic of the organ-fixing instrument pertaining to the present invention is the ability provided to insert a thread into the sheath, link the tip thereof to the tip of the sheath, and draw the suturing thread catch in a transverse direction by pulling the base side of the thread. Drawing of the suturing thread catch in a transverse direction is thereby accomplished through a simple means.

A structural characteristic of the gastric fistula catheter set pertaining to the present invention is that the gastric fistula catheter comprises the aforementioned organ-fixing instrument, and a bumper part attached to the tip of the tube, and once the tube and the bumper part are extended and inserted into the sheath and the bumper part is made to project from the tip aperture of the sheath, the suturing thread catch can be drawn in a transverse direction by restoring the tube and the bumper part to their original state.

Installation of a gastric fistula catheter and fixation of the stomach can thereby be performed simultaneously, and the procedure for installing a gastric fistula catheter in a patient is also facilitated. Elongation of the gastric fistula catheter can also be performed, for example, by inserting a rod-shaped extender from the tip aperture of the tube and pressing the bumper part at the tip thereof toward the tip, and restoration of the gastric fistula catheter to its original state can be performed by withdrawing the extender from within the gastric fistula catheter. In this instance, the suturing thread catch can be drawn in a transverse direction by pressing the tip of the suturing thread catch toward the base part only by the length that the tube and the tube-side portion of the bumper part are extended (the amount of contraction produced by releasing the pulling force).

A structural characteristic of the installation method pertaining to the present invention is the provision of a process in which a guide wire is passed from the skin surface of a patient to a predetermined organ in the body; a process in which a sheath of an organ-fixing instrument is passed, via the guide wire, from the skin surface of a patient to a predetermined organ in the body; a process in which a dilator is inserted into the organ-fixing instrument to spread the sheath and the holes provided in the body of the patient in a transverse direction; a process in which a gastric fistula catheter is elongated and inserted into the organ-fixing instrument wherein the sheath is spread in a transverse direction, and a bumper part is made to project from the tip aperture of the sheath; a process in which a suturing thread catch is drawn in a transverse direction by restoring the gastric fistula catheter to its original state; a process in which a pair of puncture needles, into which a suturing thread has been passed from a pair of puncture needle holes in a body surface installation part, is in each case made to puncture and pass through the suturing thread catch; a process in which the guide wire is withdrawn; a process in which the pair of puncture needles and the organ-fixing instrument are pulled toward the skin surface of the patient and the tip of the pair of suturing threads is in each case wrapped onto the suturing thread catch, and the tip portion of the pair of suturing threads is also drawn to the outside of the body together with the sheath; and a process in which the tips of each suturing thread in the pair of suturing threads are tied to each other.

The sheath of the organ-fixing instrument is thereby passed, via a guide wire, from the skin surface of a patient to an organ in the body, and the procedure for inserting the sheath into the body of the patient is thus facilitated. Additionally, once the sheath inserted into the body of the patient and the holes provided in the body of the patient are spread in a transverse direction by the dilator, an elongated gastric fistula catheter is inserted into the organ-fixing instrument, and the procedure for installing the gastric fistula catheter is thus facilitated. In addition, the bumper part restores the gastric fistula catheter located in the organ to its original state, and the suturing thread catch assumes a state drawn in a transverse direction, and the procedure for drawing the suturing thread catch in a transverse direction is thus facilitated. The tips of suturing threads then puncture the suturing thread catch and are wrapped onto the suturing thread catch simply by passing a pair of puncture needles, through each of which a suturing thread has been passed, into a pair of needle through-holes in the body surface installation part, and the procedure for wrapping the suturing thread onto the suturing thread catch is thereby facilitated, and the suturing thread is also wrapped reliably onto the suturing thread catch.

### DETAILED DESCRIPTION OF THE DRAWINGS

In the drawings, the following symbols are used:
10). organ-fixing instrument, 11) body surface installation part, 12) sheath, 13) through-hole, 14,15) needle through-hole, 16) covering, 17) reinforcing part, 18) suturing thread catch, 18a,18b) skeletal part, 21) dilator, 22) gastric fistula catheter, 24) tube, 25) bumper, 31,32) puncture needle, 33a,33b) suturing thread, A) abdominal wall, B) stomach wall.

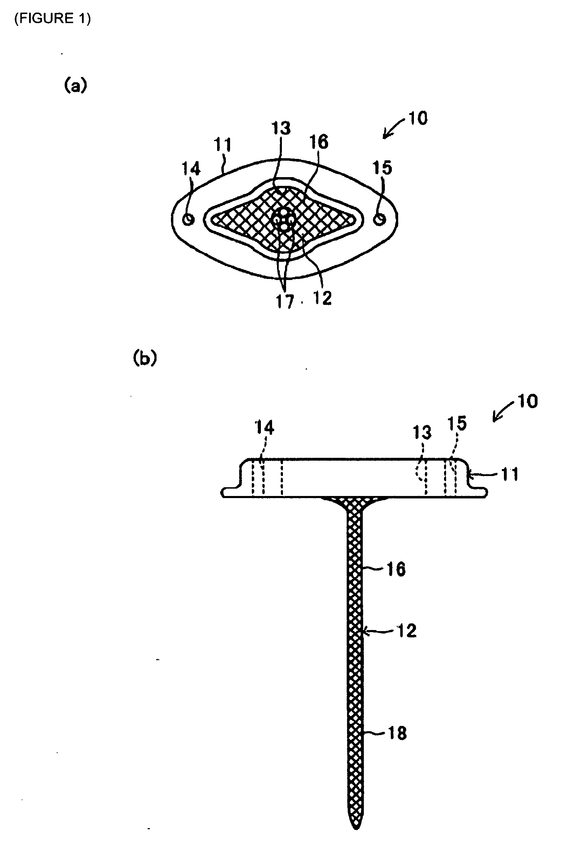

One preferred embodiment of the present invention is described hereafter using drawings. Figure 1 illustrates an organ-fixing instrument 10 pertaining to the present preferred embodiment. The organ-fixing instrument 10 comprises a body surface installation part 11 and a sheath 12 linked to the body surface installation part 11. The body surface installation part 11 I comprises a compact of a resin material; its planar aspect is formed as a nearly elliptical, thick sheet; and in a planar aspect passing from the top surface to the bottom surface there is formed an approximately rhomboid, large through-hole 13. On both sides of the lengthwise direction of said through-hole 13, small, circular needle through-holes 14, 15 are formed so as to enclose the through-hole 13 and assume a fixed distance from the through-hole 13. The needle through-holes 14, 15 are parallel to each other and pass through the body surface installation part 11 from the top surface to the bottom surface.

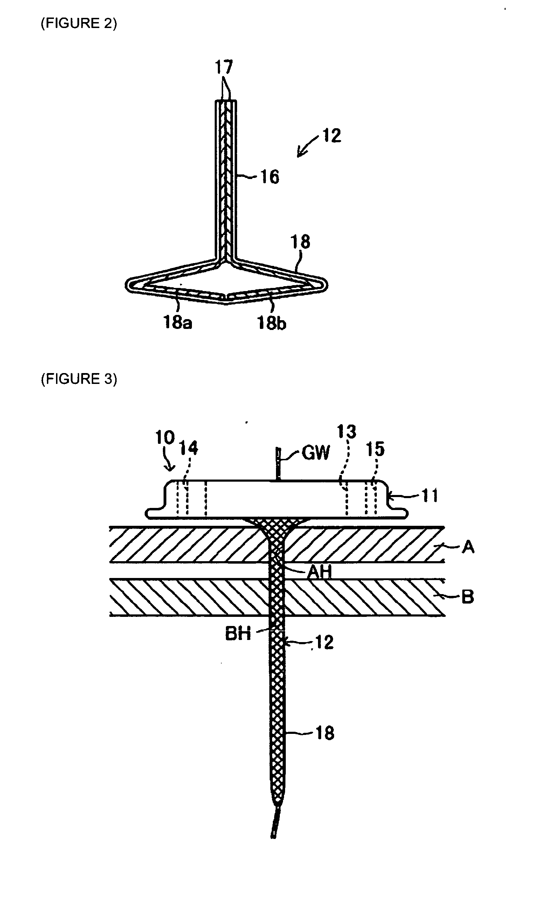

The sheath 12 comprises a covering 16 comprising a transversely elastic body made from a webbed tube incorporating a laminate resin and a webbed locking part made from a resin; a plurality of reinforcing parts 17 formed on the inner circumferential surface of the base portion (body surface installation part 11 side) of the covering 16; and a pair of skeletal parts 18a, 18b (see Figure 2) formed on the inner circumferential surface of the tip portion of the covering 16. The upper end of the covering 16 is affixed to the inner circumferential surface of the through-hole 13 in the body surface installation part 11. The reinforcing parts 17 comprise a plurality, for example four, of slender, rod-shaped parts disposed with maintenance of a fixed interval in the circumferential direction of the inner circumferential surface of the covering 16, and said reinforcing parts 17 are formed so as to extend from the base-end region of the covering 16 to the approximate center of the covering 16. The skeletal parts 18a, 18b comprise rods in which the central part of each rod is formed in a thin-walled part, and in which said thin-walled part is bendable at the center, and said skeletal parts 18a, 18b are provided opposite to each other at the tip region on the inner surface of the covering 16.

The pair of skeletal parts 18a, 18b are compressed in an axial direction, and as shown in Figure 2, the central thin-walled portion is thus bent so as to project outward. At such time, the covering 16 is pressed and spread by the pair of skeletal parts 18a, 18b and thus spreads while still covering the skeletal parts 18a, 18b. A suturing thread catch 18 is comprised by the tip portion of the covering 16 and the pair of skeletal parts 18a, 18b. The suturing thread catch 18 is designed such that its spreading direction is the same as the direction in which the needle through-holes 14, 15 of the body surface installation part 11 are provided. When the plurality of reinforcing parts 17 are brought into proximity to each other, and the skeletal parts 18a, 18b are at the same time brought into proximity to each other so that the covering 16 approaches the central axis in contracting fashion, the sheath 12 assumes a long, slender rod shape; and when the respective reinforcing parts 17 are moved apart from each other and the skeletal parts 18a, 18b are at the same time moved apart from each other so that the covering 16 spreads transversely, the sheath 12 assumes a cylindrical shape.

When an organ-fixing instrument 10 structured in this manner is used, for example, when the abdominal wall and stomach wall of a patient are sutured together to fix the stomach to the abdominal wall, first, as shown in Figure 3, a guide wire GW is inserted into the abdominal wall A and the stomach wall B, and this guide wire GW is used to insert the sheath 12 of the organ-fixing instrument 10 into the hole AH in the abdominal wall A and the hole BH in the stomach wall B. When the guide wire GW is inserted, first, the body of the patient is punctured with a cannula (not illustrated) comprising a cylindrical puncture needle, and the tip thereof is brought to the inside of the stomach wall B. Next, the guide wire GW is passed through the cannula, and the tip of the guide wire GW is inserted at the inside of the stomach wall B. Then, with the guide wire GW remaining in the body of the patient, the cannula is withdrawn from the body of the patient.

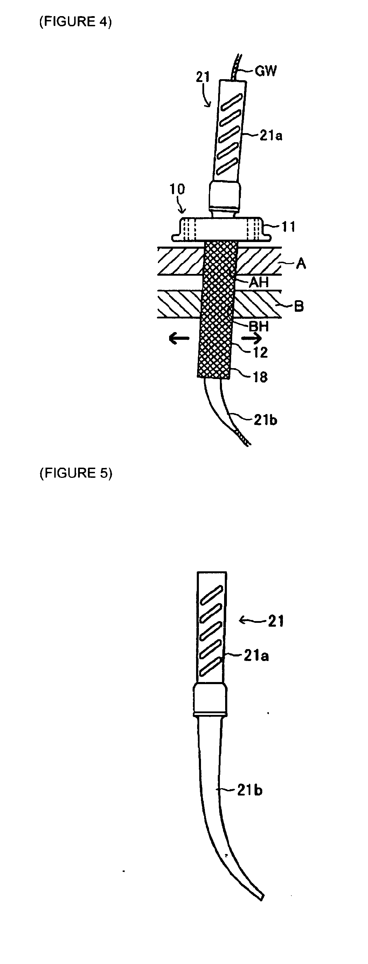

Next, the extracorporeal portion of the guide wire GW is passed from the aperture at the tip of the sheath 12 to the interior and then projected to the outside from the through-hole 13 in the body surface installation part 11. The organ-fixing instrument 10 is then pressed into the body along the guide wire GW, the tip portion of the sheath 12 is positioned on the inside of the stomach wall B, and the base portion of the sheath 12 is positioned at the hole AH in the abdominal wall A and the hole BH in the stomach wall B. These procedures create the state shown in Figure 3. Next, as shown in Figure 4, a dilator 21 is inserted from the through-hole 13 in the body surface installation part 11 toward the sheath 12, and the sheath 12, hole AH, and hole BH are spread transversely, as indicated by the arrow.

As shown in Figure 5, the dilator 21 comprises a clasp 21a and a transverse expander 21b linked to the clasp 21a; the transverse expander 21b becomes progressively narrower from the base to the tip and comprises a rod-shaped body bent into an arcuate shape at the tip. A through-hole (not illustrated) allowing insertion of the guide wire GW is also formed from the top end of the clasp 21a (top end in state shown in Figure 4 and Figure 5) to the tip of the transverse expander 21b. Consequently, the dilator 21 can be moved along the guide wire GW with the extracorporeal portion of the guide wire GW in a state passing from the tip aperture of the dilator 21 to the interior and projecting to the exterior from the top end of the clasp 21a.

Insertion of the transverse expander 21 b into the sheath 12 from the through-hole 13 of the body surface installation part 11 expands the diameter of the sheath 12 and also allows the hole AH in the abdominal wall A and the hole BH in the stomach wall B to spread. At such time, the transverse expander 21 b in the dilator 21 enters the sheath 12 along the lengthwise direction of the reinforcing parts 17, thus allowing smooth insertion. Each reinforcing part 17 spreads to nearly equal intervals, with no misalignment to the location of the hole AH in the abdominal wall A and the hole BH in the stomach wall B. It is preferable to provide a lubricating coating to the surface of the dilator 21.

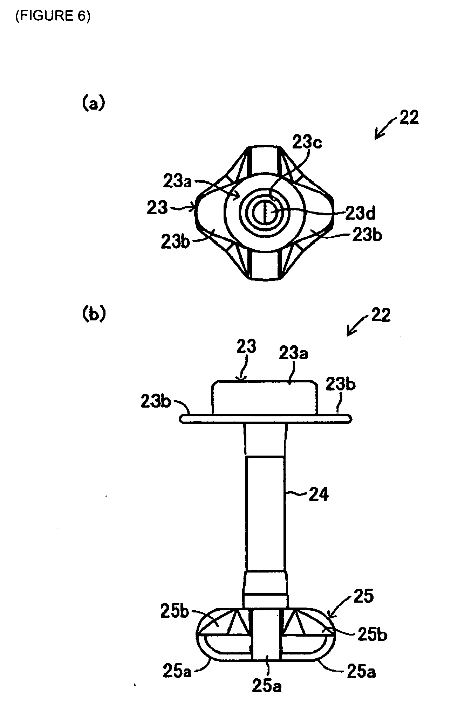

Next, once the dilator 21 is withdrawn from the organ-fixing instrument 10, the gastric fistula catheter 22 is installed in the organ-fixing instrument 10. The gastric fistula catheter 22 is structured as shown in Figure 6. Specifically, the gastric fistula catheter 22 comprises an external holder 23, a tube 24 linked to the center of the bottom endface of the external holder 23, and a bumper 25 linked to the tip of the tube 24, with each element made from polyurethane. The external holder 23 comprises an insertion aperture 23a formed as a somewhat thick ring, and a pair of protrusions 23b protruding outward from both sides of the outer circumferential bottom end of the insertion aperture 23a.

An insertion hole 23c passing from top to bottom is formed at the center of the insertion aperture 23a, and a check valve 23d with a slit formed centrally is provided on the inner circumferential surface of the insertion hole 23c. The shape of the external holder 23 as seen from above is the same as the shape of the through-hole 13 in the body surface installation part 11 as seen from above. The external holder 23 can thus be arranged to face the through-hole 13 and thereby allow insertion.

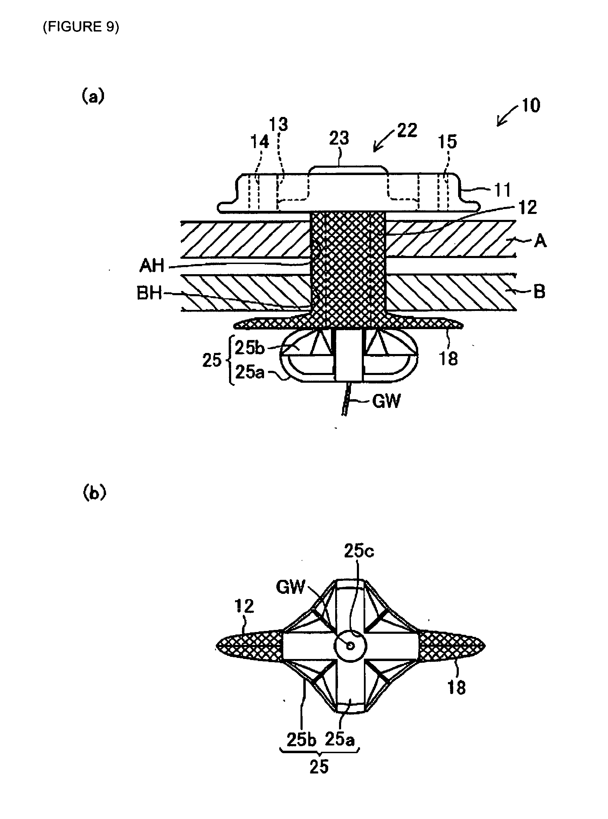

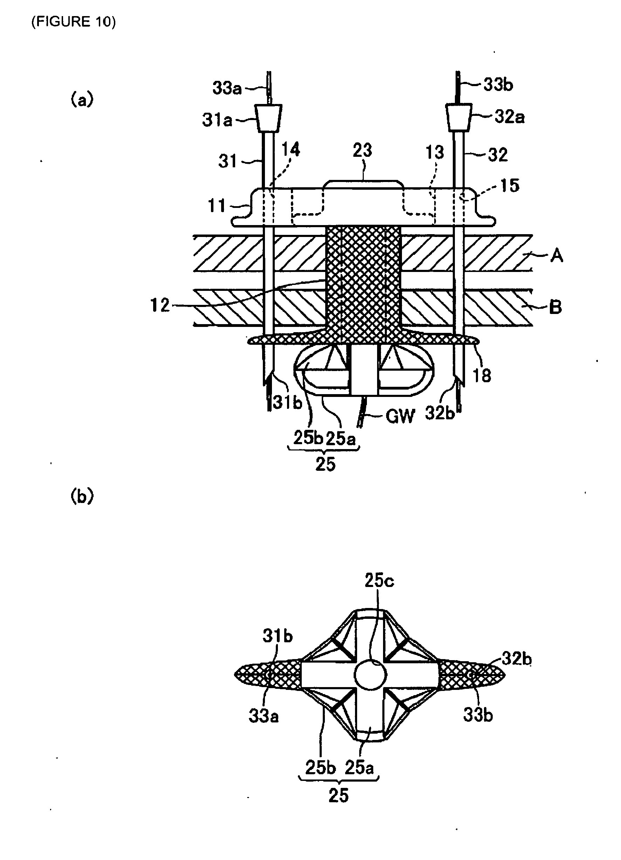

The tube 24 comprises a long-slender cylinder wherein there is formed a fluid flow path (not illustrated) for transport of a nutritional agent, fluid food, or other such fluid, and the top end of the fluid flow path communicates with the insertion hole 23c in the external holder 23. The bumper 25 is connected to the bottom end of the tube 24. The bumper 25 comprises four band-shaped linkages 25a extending in every direction from the edges of the bottom end aperture of the tube 24, and four linkage film parts 25b provided between the top portion of each linkage 25a, and the tips of each linkage 25a are linked to each other. A hole 25c (see Figure 9(b) and Figure 10(b)) is formed at the part of each linkage 25a where the tips are linked. The tube 24 and bumper 25 are each elastic.

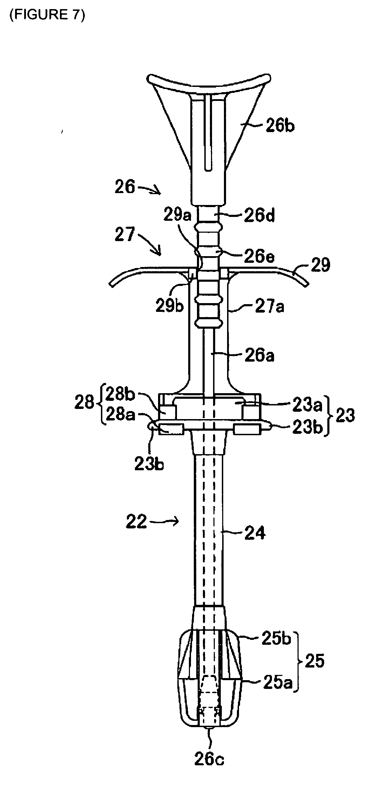

The procedure for installing the gastric fistula catheter 22 in the organ-fixing instrument 10, as shown in Figure 7, is carried out with the organ-fixing instrument 10 in an extended state, using an extender comprising a rod 26 and a locking member 27. The rod 26 is provided with a rod body 26a made from a stainless steel shaft, and with a plastic clasp 26b. A plastic insertion part 26c is attached at the tip of the rod body 26a. The insertion part 26c is formed with a stepped shape having a major diameter at its central portion in a vertical orientation, the diameter of a narrow part of the tip is smaller than the inner diameter of the hole 25c in the bumper 25, and the diameter of the central major diameter part is larger than the inner diameter of the hole 25c in the bumper 25.

Consequently, when the rod body 26a of the rod 26 is inserted toward the inside from the insertion hole 23c of the gastric fistula catheter 22, the narrow-diameter part of the tip of the insertion part 26c enters the hole 25c, and the large-diameter part of the center of the insertion part 26c assumes a state of engagement with the part where the tip of each linkage 25a is linked. As a result, when the rod 26 is pressed into the gastric fistula catheter 22, the tube 24 extends in a lengthwise direction, and the bumper 25 narrows and to some extent elongates. Additionally, on the top portion of the rod 26, a cylindrical part 26d structured integrally with the clasp 26b is formed so as to cover the outer circumferential surface of the rod body 26a, and on the outer circumferential surface thereof, a plurality of catch parts 26e comprising a ring-shaped projection are formed at vertical intervals. A through-hole (not illustrated) allowing passage of the guide wire GW from the base to the tip of the rod 26 is also provided.

The locking member 27 is formed by working a stainless steel sheet and is provided with a lower locking part 28 and an upper locking part 29. The lower locking part 28 and the upper locking part 29 are linked by a square, planar linking tab 27a elongated in a vertical direction. The lower locking part 28 comprises a retaining tab 28a which is approximately U-shaped in horizontal perspective and which, from the bottom end of the linking tab 27a, is formed perpendicular to the linking tab 27a and oriented horizontally to the near side of the illustration; and a pair of flanges 28b which, from both sides of the bottom end side regions of the linking tab 27a, are perpendicular to the linking tab 27a and parallel to the retaining tab 28a at an interval maintained from the retaining tab 28a. The inner portion of the approximately U-shaped retaining tab 28a is formed with a size able to admit the base of the tube 24, and the interval between the retaining tab 28a and flanges 28b is a size able to enclose the pair of protrusions 23b.

The tip of the retaining tab 28a is curved upward to prevent release of engagement with the pair of protrusions 23b. The upper locking part 29, from the top end of the linking tab 27a, is formed perpendicular to the linking tab 27a and oriented horizontally to the near side of the illustration, and comprises a horizontal portion extending toward both sides of the linking tab 27a. Individual catch parts 26e and engagable catch recesses 29a are formed at the central near portion of the upper locking part 29, and a pair of projections 29b intended to prevent release of engagement with the catch parts 26e project downward at both side regions of the catch recesses 29a.

When the gastric fistula catheter 22 is extended using the extender, first, the rod body 26a of the rod 26 is inserted into the gastric fistula catheter 22, and the tip of the insertion part 26c is inserted into the hole 25c in the bumper 25. Next, with the protrusions 23b on the gastric fistula catheter 22 held between the retaining tab 28a and the flanges 28b, and the rod body 26a positioned inside the catch recesses 29a, the locking member 27 is applied to the gastric fistula catheter 22 and the rod 26. Next, with the clasp 26b pressed, the locking member 27 is pulled upward, and the edge of the catch recesses 29a is made to engage a predetermined catch part 26e. Thereby, as shown in Figure 7, the bumper 25 is stretched straight, the bumper 25 narrows, and the tube 24 and bumper 25 assume a shape nearly that of a single rod. The tube 24 is also elongated.

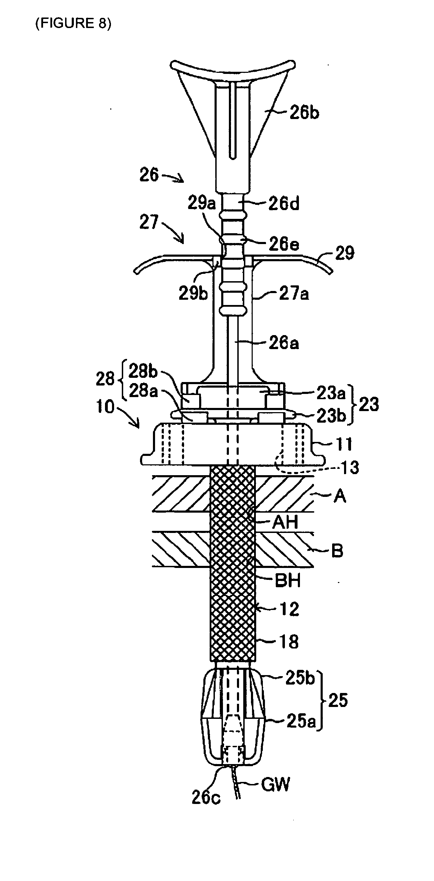

The gastric fistula catheter 22 in a state of extension by the extender, as shown in Figure 7, is inserted into the organ-fixing instrument 10 which is itself inserted in the hole AH in the abdominal wall A and the hole BH in the stomach wall B, and the gastric fistula catheter 22 assumes the state in Figure 8. In this instance, the procedure is performed by passing the guide wire GW into the through-hole of the rod 26. Next, the catch recesses 29a are released from the catch parts 26e where the bumper 25 is admitted to the inside of the stomach wall B; the retaining tab 28a and flanges 28b are also released from the protrusions 23b, and the locking member 27 is released from the gastric fistula catheter 22. The rod 26 is also withdrawn from the gastric fistula catheter 22.

Thus, as shown in Figure 9, the tube 24 of the gastric fistula catheter 22 contracts in the axial direction, and the bumper 25 is restored by elasticity to its original, nearly spherical shape. Consequently, the tip of the sheath 12 of the organ-fixing instrument 10 is compressed upward by the gastric fistula catheter 22, and the suturing thread catch 18 assumes a pendant state along the lengthwise direction of the body surface installation part 11, which is part of the circumferential direction. At such time, both ends of the suturing thread catch 18 project further outward than the location directly under the needle through-holes 14, 15 in the body surface installation part 11 and the outer circumferential part of the bumper 25. In this instance, as shown in Figure 9(b), alignment arises between the orientation along two linkages 25a of the four linkages 25a on the gastric fistula catheter 22 and the suturing thread catch 18, which facilitates application of force by the bumper 25 to the suturing thread catch 18.

Next, as shown in Figure 10, the abdominal wall A and stomach wall B are sutured using a pair of puncture needles 31, 32 and a pair of suturing threads 33a, 33b. The puncture needles 31, 32 comprise a metal, cylindrical body formed with a through-hole (not illustrated) inside, and clasps 31a, 32a are attached to the base. The clasps 31a, 32a are formed as a cylindrical shape with a large diameter toward the top and a small diameter toward the bottom, and a guide hole communicating with the through-hole is formed inside. The tip of the puncture needles 31, 32 is also cut across an oblique direction and thus formed such that apertures 31b, 32b are visible from a horizontal orientation.

One puncture needle 31 is passed through a needle through-hole 14 in the body surface installation part 11, and the tip thereof is passed through the abdominal wall A, stomach wall B, and suturing thread catch 18; while the other puncture needle 32 is passed through another needle through-hole 15 in the body surface installation part 11, and the tip thereof is passed through the abdominal wall A, stomach wall B, and suturing thread catch 18. In this instance, a stopper in direct contact with the body surface installation part 11 is provided movably at a predetermined part of the puncture needles 31, 32, and the insertion length of the puncture needles 31, 32 may be adjusted. One suturing thread 33a is passed into one puncture needle 31, and the tip thereof is projected toward the inside of the stomach wall B; while another suturing thread 33b is passed into the other puncture needle 32, and the tip thereof is projected toward the inside of the stomach wall B. These procedures produce the state shown in Figure 10. The shape of the tip of the suturing threads 33a, 33b may be hooked in each case, or the central part of a rod-shaped member may be linked to the tip of the thread to create a shape able to wrap reliably onto the suturing thread catch 18.

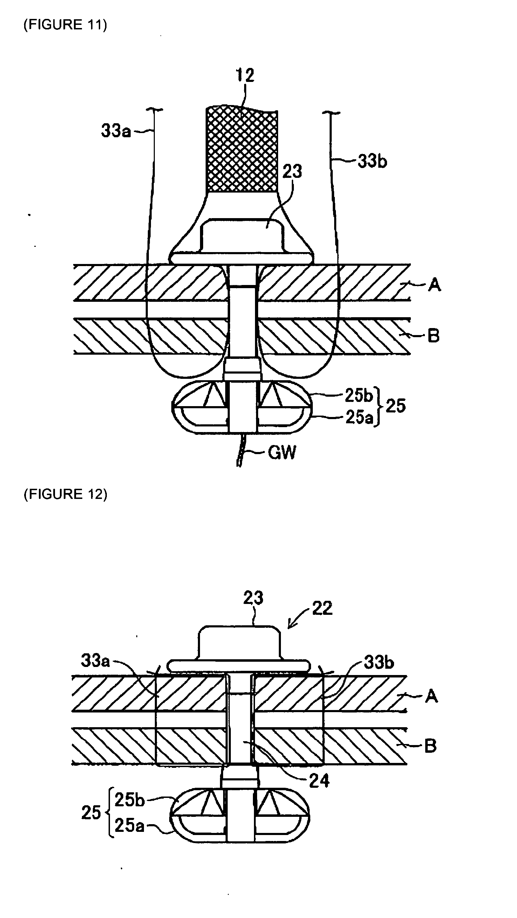

Next, the puncture needles 31, 32 are pulled out toward the outside of the body, and the suturing threads 33a, 33b are pulled lightly to wrap the tips of the suturing threads 33a, 33b onto the suturing thread catch 18. The organ-fixing instrument 10 is then pulled out of the body. Thereby, as shown in Figure 11, the sheath 12 of the organ-fixing instrument 10 passes between the circumferential surface of the hole AH in the abdominal wall A and the hole BH in the stomach wall B and the outer circumferential surface of the tube 24, and the external holder 23 is thereby passed to the inside, and [the sheath 12 is] thus pulled out of the body. The base-side portion of the suturing threads 33a, 33b is also fed out sequentially, and the tip thereof is passed from one hole BH to the other hole AH together with the sheath 12 and is thus pulled out of the body.

At such time, the reinforcing parts 17 or skeletal parts 18a, 18b are bent along the gastric fistula catheter 22 together with the covering 16. In this state, the guide wire GW is withdrawn from the body, and the suturing thread catch 18 is also released from the suturing threads 33a, 33b. The guide wire GW may also be withdrawn before the organ-fixing instrument 10 and puncture needles 31, 32 are pulled out of the body. Thereby, once the suturing threads 33a, 33b have each entered the inside of the stomach wall B from the area punctured by the puncture needles 31, 32, the suturing threads 33a, 33b pass through one hole BH and the other hole AH and then extend outside the body.

The extending portion of both sides of the suturing threads 33a, 33b is then cut to form a predetermined length, and both ends are tied to achieve the state shown in Figure 12 and complete suturing. Furthermore, the organ-fixing instrument 10 may be given a peel-off form to split the body surface installation part 11 and sheath 12 in half. The procedure of withdrawing the organ-fixing instrument 10 from the body is thereby facilitated.

As described above, in the organ-fixing instrument 10, a through-hole 13 communicating with a sheath 12, and needle through-holes 14, 15 allowing passage of puncture needles 31, 32 are formed in the body surface installation part 11, and a suturing thread catch 18 projected in a transverse direction by compression applied in an axial direction is provided toward the tip of the sheath 12. Consequently, the sheath 12 of the organ-fixing instrument 10 can be inserted into both one hole AH and another hole BH, and the suturing thread catch 18 can be placed in a state projecting transversely inside the stomach wall B. Then, when the suturing threads 33a, 33b are each passed by way of the puncture needles 31, 32 through the abdominal wall A and stomach wall B and the tips thereof are wrapped onto the suturing thread catch 18, the suturing threads 33a, 33b are each passed by way of the sheath 12 out of the holes made by the puncture needles 31, 32 and through toward the inside of the stomach wall B, are passed through one hole BH and another hole AH, and assume an approximately U-shaped, continuous form.

As a result, by tying both sides of each of the suturing threads 33a, 33b located on the skin side of the patient, the abdominal wall A and stomach wall B can be fixed by the suturing threads 33a, 33b. Additionally, because the sheath 12 of the organ-fixing instrument 10 is formed with a transversely elastic cylindrical shape, the sheath 12 assumes a slender, rod shape when inserted into one hole AH and another hole BH, and once the sheath 12 is inserted into one hole AH and another hole BH, the dilator 21 can be inserted into the sheath 12 to enlarge the diameter of the sheath 12 and the holes AH, BH. The procedure for installing the gastric fistula catheter 22 is thereby facilitated.

In an instance where the dilator 21 is used to enlarge the diameter of the holes AH, BH, and the dilator 21 is inserted directly into the holes AH, BH, the region near the circumferential surface of the holes AH, BH becomes pressed into the body. However, as described above, use of the sheath 12 tends to transmit the force of the dilator 21 horizontally, and the force pressing the region near the circumferential surface of the holes AH, BH into the body is diminished. Consequently, adequate transverse expansion of the holes AH, BH is made possible, even if the inserted length of the dilator 21 is short.

In the organ-fixing instrument 10, the sheath 12 comprises a cylindrical covering 16 comprising a tube incorporating a laminate resin and a webbed locking part made from a resin, a plurality of reinforcing parts 17 provided at the base end part of the covering 16 and extending in an axial direction, and skeletal parts 18a, 18b with a hinged construction provided toward the tip of the covering 16. A suturing thread catch 18 is also formed at both the tip part of the covering 16 and the skeletal parts 18a, 18b. Structuring the body of the sheath 12 in this manner with a covering 16 equipped with a webbed locking part facilitates wrapping of the suturing threads 33a, 33b onto the suturing thread catch 18.

Provision of the reinforcing parts 17 also greatly increases the strength of the base end part located at the holes AH, BH when the sheath 12 is inserted in the holes AH, BH. Any occurrence of misalignment with the respective holes AH, BH is thus prevented. Use of the projection of the suturing thread catch 18 and the skeletal parts 18a, 18b also allows construction of the suturing thread catch 18 with a simple structure. Additionally, the present preferred embodiment allows simultaneous performance of a procedure to install the gastric fistula catheter 22 in the holes AH, BH and a procedure to suture the abdominal wall A and stomach wall B and also facilitates the procedure to install the gastric fistula catheter 22 in the holes AH, BH.

The organ-fixing instrument pertaining to the present invention is also not limited to the preferred embodiment described above, which can be implemented with changes as suitable. For example, in the preferred embodiment described above, a rod formed with the center part as a thin-walled part is used as a skeletal part 18a, 18b of the suturing thread catch 18, but a skeletal part is not limited thereto, and another acceptable item is one equipped with a hinged structure which is projected in a direction perpendicular to the axial direction by application of a force tending to bring both ends closer along the axial direction. An item is also acceptable wherein a thread is inserted into the sheath 12 and the tip thereof is linked to the sheath 12, and the suturing thread catch 18 can then be pulled in a transverse direction by pulling the base side of the thread.

The reinforcing parts 17 of the sheath 12 may also be omitted, and the number thereof may be established as desired. For example, a large number of very fine parts may be provided. Depth marks may also be provided on the surface of the sheath 12. Other needle through-holes additional to the needle through-holes 14, 15 described above may also be provided with different spacing. Additionally, in the preferred embodiment described above, the covering 16 of the sheath 12 comprises a webbed tube incorporating a laminate resin and a webbed locking part made from a resin, but the covering 16 may also be structured by a mesh-form tube comprising a mesh made from a resin. Aspects such as the shape of the body surface installation part 11 or the gastric fistula catheter 22 may also be changed as appropriate.

## Claims

1. An organ-fixing instrument equipped with a transversely elastic cylindrical sheath and a body surface installation part attached to the base end part of the sheath, and used when fixing a predetermined organ to the skin-side wall area with suturing thread in the body of a patient; **characterized in that** the body surface installation part is equipped with a through-hole communicating with the sheath, and with a pair of needle through-holes enclosing the through-hole and formed at both sides of the through-hole and allowing passage of a puncture needle approximately parallel to the sheath; and wherein the sheath is equipped with a suturing thread catch which is provided at the tip side of the sheath and said suturing thread catch is projected in a transverse direction by application of a force which brings both ends of the sheath closer to each other along an axial direction and said suturing thread catch is deformed when the puncture needles pass into the pair of needle through-holes until said puncture needles reach a puncturable location.

2. The organ-fixing instrument according to Claim 1, wherein the body of the sheath is structured with a covering part comprising a tube made from a resin and equipped with a webbed locking part, and the suturing thread catch is structured by the provision of a skeletal part with a hinged structure on the tip side of the covering part.

3. The organ-fixing instrument according to Claim 1, or Claim 2, wherein a reinforcing part extending along an axial direction is provided at the base end side portion of the covering part.

4. The organ-fixing instrument according to any one of Claim 1 through Claim 3, wherein the suturing thread catch is projected into a transverse direction by inserting a thread into the sheath thereby linking the tip of the thread to the tip of the sheath, and pulling the base side of the thread.

5. A gastric fistula catheter set comprising:
an organ-fixing instrument according to any one of Claim 1 through Claim 4, and
a gastric fistula catheter comprising an external holder, a tube linked to the center of the bottom endface of the external holder, and a bumper part attached to the tip of the tube.

6. The gastric fistula catheter set according to claim 5, wherein the bumper part comprises four band-shaped linkages extending in four directions from the edges of a bottom end aperture of the tube, and four linkage film parts provided between a top portion of each linkage, wherein the tips of each linkage are linked to each other.
